# EUROPEAN PATENT SPECIFICATION

(11) **EP 2 438 922 B1**
(45) Date of publication and mention of the grant of the patent: **26.08.2020**
(21) Application number: 09845189.1
(22) Date of filing: 26.05.2009
(51) Int. Cl.: A61K 33/42, A61K 47/42, A61K 47/36, A61K 47/18, A61K 47/10, A61K 9/14, A61Q 19/08, A61K 8/24, A61K 8/02, A61K 45/06, A61K 31/047, A61K 31/19, A61K 31/70, A61K 31/715

(54) **COLLAGEN PRODUCTION ENHANCER**
VERSTÄRKER FÜR KOLLAGENPRODUKTION
PROMOTEUR DE LA PRODUCTION DE COLLAGÈNE

(43) Date of publication of application: 11.04.2012
(73) Proprietor: SofSera Corporation, Tokyo 160-0022 (JP)
(72) Inventor: KOGAI Yasumichi, Tokyo 160-0022 (JP); KAWABE Karl Kazushige, Tokyo 160-0022 (JP)
(74) Representative: Hoffmann Eitle
(86) International application number: PCT/JP2009/059631
(87) International publication number: WO 2010/137122

(56) References cited:
- EP-A1- 1 808 411
- WO-A1-2006/030782
- WO-A2-2006/050368
- JP-A- 11 029 486
- JP-A- 2001 302 454
- JP-A- 2007 282 515
- JP-A- 2009 101 147
- US-A- 5 204 382
- US-A1- 2008 160 088
- ROBERT VOIGTS ET AL: "Dispersion of Calcium Hydroxylapatite Accumulations in the Skin: Animal Studies and Clinical Practices", DERMATOLOGIC SURGERY, vol. 36, no. s1, 1 May 2010 (2010-05-01), pages 798-803, XP055069465, ISSN: 1076-0512, DOI: 10.1111/j.1524-4725.2010.01567.x
- BERLIN ET AL: "Calcium Hydroxylapatite for Facial Rejuvenation", SEMINARS IN CUTANEOUS MEDICINE AND SURGERY, W.B. SAUNDERS, PHILADELPHIA, US, vol. 25, no. 3, 1 September 2006 (2006-09-01), pages 132-137, XP005703027, ISSN: 1085-5629, DOI: 10.1016/J.SDER.2006.06.005
- KYLE M. COLEMAN ET AL: "Neocollagenesis after Injection of Calcium Hydroxylapatite Composition in a Canine Model", DERMATOLOGIC SURGERY, vol. 34, no. s1, 1 June 2008 (2008-06-01), pages S53-S55, XP055069455, ISSN: 1076-0512, DOI: 10.1111/j.1524-4725.2008.34243.x
- BIGI A ET AL.: 'Human osteoblast responce to pulsed laser deposited calcium phosphate coatings' BIOMATERIALS vol. 26, 2005, pages 2381 - 2389, XP027768120
- YU HYE-SUN ET AL.: 'Apatite-mineralized polycaprolactone nanofibrous web as a bone tissue regeneration substrate' JOURNAL OF BIOMEDICAL MATERIALS RESEARCH PART A vol. 88, no. 3, 20 March 2009, pages 747 - 754, XP008148654

## Description

### TECHNICAL FIELD

The present invention relates to the cosmetic use of a composition for enhancing collagen production or the use of a collagen production enhancer as addendum for a cosmetic, as defined in the claims.

### BACKGROUND ART

Collagen is present in almost every organ of a human. In particular, it is known that about 70% of dry mass of dermis is collagen. It is also known that collagen is useful for maintaining functions such as elasticity, flexibility, or moisture retention property of skin and also has an influence on maintaining cell morphology in normal state as well as metabolism or adhesion.

Skin is constantly exposed to an outside environment, and with a loss of resilience or vibrance according to aging, aging phenomena such as wrinkles or saggings occurs. It is believed that such phenomena are caused by a decreasing amount of collagen present in skin according to aging process . To eliminate wrinkles or saggings, various collagen production enhancers wherein a material having an activity of promoting collagen production are reported (JP-A-2008-260747). Conventionally, it is known that collagen is produced by fibroblast present in dermis and the enhancer promotes the production of collagen by fibroblast.

K.M. Coleman et al., Dermatol. Surg., 34, S53-S55 (2008) relates to neocollagenesis after injection of calcium hydroxylapatite composition in a canine model.

### DISCLOSURE OF INVENTION

### Problems to be Solved by the Invention

An object of the invention is to provide a novel collagen production enhancer for increasing the amount of collagen contained in skin tissues.

### Means for Solving the Problems

The present invention is directed to cosmetic use of a composition comprising
(i) calcium phosphate fine particles as effective ingredients, wherein
   - the average particle diameter of the calcium phosphate fine particles is 20-250 nm,
   - the calcium phosphate fine particles are a sintered body wherein the half width at d = 2.814, as measured by X ray diffraction (XRD), is ≤ 0.8, and
(ii) at least one material selected from alcohols, sugars, proteins, amino acids, water soluble vitamins, fat soluble vitamins, lipids, mucosaccharides, and a surface-active agent, for enhancing collagen production.

Also, the present invention is directed to the use of a collagen production enhancer comprising
(i) calcium phosphate fine particles as effective ingredients, wherein
   - the average particle diameter of the calcium phosphate fine particles is 20-250 nm,
   - the calcium phosphate fine particles are a sintered body wherein the half width at d = 2.814, as measured by X ray diffraction (XRD), is ≤ 0.8, and
(ii) at least one material selected from alcohols, sugars, proteins, amino acids, water soluble vitamins, fat soluble vitamins, lipids, mucosaccharides, and a surface-active agent,
as addendum for a cosmetic.

Preferred embodiments of the invention are as defined in the appended dependent claims and/or in the following detailed description.

### Effects of the Invention

The present invention exhibits an effect of enhancing collagen production via action on fibroblast. It also exhibits an effect of ameliorating aging phenomena such as wrinkles and saggings and reconstructing damaged collagen fibers in tissues to convert them into normal tissues.

According to the invention, the average particle diameter of calcium phosphate fine particles is very small, and therefore an effect of ameliorating skin texture exhibited by coating them on skin surface.

In a preferred embodiment of the invention the sintered body is produced by a method including the steps of mixing primary particles containing calcium phosphate with a fusion preventive agent, and sintering the mixed particles obtained from the mixing step by exposing them to a sintering temperature in the range of 100-1,800°C. In this case the calcium phosphate fine particles are maintained in primary particle state as they are not fused to each other even by sintering, thus a sintered body can be obtained while maintaining the particles in the small size, and therefore a strong effect of enhancing collagen production is exhibited.

Also, an effect of promoting rapid penetration into dermis, and an astringent, moisturizing, and anti-inflammatory effect are exhibited by containing the materials (ii) described above.

### BRIEF DESCRIPTION OF DRAWINGS

FIG. 1 is a drawing which illustrates the state of skin surface of test subject No. 1; and
FIG. 2 is a drawing which illustrates the state of skin surface of test subject No. 2.

### BEST MODE(S) FOR CARRYING OUT THE INVENTION

In the use of a collagen production enhancer as addendum for a cosmetic according to the invention, the collagen production enhancer contains calcium phosphate fine particles. In the cosmetic use of a composition for enhancing collagen production according to the invention, the composition contains calcium phosphate fine particles. It contains at least one material selected from alcohols, sugars, proteins, amino acids, water soluble vitamins, fat soluble vitamins, lipids, mucosaccharides, and surface-active agents. Examples of the calcium phosphate fine particles according to the most preferred embodiment of the invention include hydroxyapatite (Ca₁₀(PO₄)₆(OH)₂), fluoro apatite (Ca₁₀(PO₄)₆F₂) and Ca₁₀(PO₄)₆C₁₂. Further, in calcium phosphate, a compound in which part of the calcium ions and/or hydroxy ions and/or phosphate ions is substituted with strontium ions, barium ions, sodium ions, bicarbonate ions, carbonate ions, fluoride ions, or chloride ions, or tricalcium phosphate (Ca₃(PO₄)₂), calcium metaphosphate (Ca(PO₃)₂), and calcium octaphosphate (OCP) may be included. Of these exemplified, hydroxyapatite is preferable. In addition, on surface of the calcium phosphate particles (particles of calcium phosphate) according to the most preferred embodiment of the invention, Ca₁₀(PO₄)₆(OH)₂ is preferably present. It is appropriate that Ca₁₀(PO₄)₆(OH)₂ is present on surface of calcium phosphate and it may be contained in an amount of 0.1 wt.-% or so relative to the total amount of calcium phosphate. More preferably, it is contained in an amount of ≥ 50 wt.-%. Further, the calcium phosphate fine particles may contain e.g. tricalcium phosphate which is generated by sintering amorphous hydroxyapatite as described below. The calcium phosphate according to the embodiment of the invention is excellent in biocompatibility with living tissues and stability in an environment of living body.

Average particle size diameter of the calcium phosphate fine particles according to the invention is 20-250 nm. By having the particle size within this range, the fine particles can penetrate a dermic layer from skin surface so that, even for a preparation for external use such as skin cosmetics, the calcium phosphate fine particles exhibit their effect on fibroblast contained in dermic layer to exhibit the function of enhancing collagen production. In particular, by having the size of 20-250 nm, an effect of easier penetration from skin surface into dermic layer can be easily exhibited as a gap between epidermal cells is 250 nm or so. The variation coefficient is preferably ≤ 20%, more preferably ≤ 18%, and still more preferably ≤ 15%. Further, the average particle diameter and variation coefficient can be calculated by measuring the particle diameter of at least 100 primary particles using an electron microscope. The "variation coefficient" is a value indicating heterogeneity of particle diameter among the particles, which is calculated as follows: standard deviation ÷ average particle diameter × (100%) . Shape of the calcium phosphate fine particles is not specifically limited, and examples thereof include a particle shape and a rod shape. In addition, when it has a rod shape, the average particle diameter is measured along the long axis diameter of the particle.

The calcium phosphate fine particles according to the invention are a sintered body. The calcium phosphate fine particles according to the most preferred embodiment of the invention is preferably a sintered body of calcium phosphate obtained by sintering (calcination) of calcium phosphate (also referred to as calcium phosphate ceramic). In particular, a sintered body obtained by dispersion calcination described below is preferable . By using a sintered body of calcium phosphate fine particles, an effect of significantly enhancing collagen production is exhibited compared to non-sintered particles. Further, the sintered body of calcium phosphate fine particles has higher crystallinity and lower solubility in a living body compared to amorphous calcium phosphate. Thus, bioactivity can be maintained in a living body for a long period of time, and therefore the effect of enhancing collagen production can be exhibited more easily for a long period of time. Sintered body of calcium phosphate particles is obtained by sintering amorphous calcium phosphate. Specifically, by carrying out sintering according to the method described below, a sintered body of calcium phosphate fine particles can be obtained, for example. It is also preferable to use highly crystalline calcium phosphate wherein the calcium phosphate fine particles have high crystallinity. The crystallinity degree of calcium phosphate can be measured by X ray diffraction (XRD). Specifically, the narrower the half width of a peak representing each crystal face, the higher the crystallinity is. As used herein, the term "highly crystalline" relating to the highly crystalline calcium phosphate of the invention indicates that the half width at d = 2.814 is ≤ 0.8 (suitably ≤ 0.5).

According to the invention, in the use of a collagen production enhancer as addendum for a cosmetic, the collagen production enhancer contains at least one material selected from alcohols, sugars, proteins, amino acids, water soluble vitamins, fat soluble vitamins, lipids, mucosaccharides, or surface-active agents. According to the invention, in the cosmetic use of a composition for enhancing collagen production, the composition contains at least one material selected from alcohols, sugars, proteins, amino acids, water soluble vitamins, fat soluble vitamins, lipids, mucosaccharides, or surface-active agents.

Examples of the alcohols include ethanol and glycerin. By containing alcohols, dispersion stability of the calcium phosphate fine particles is improved and rapid penetration into a dermic layer is promoted. Further, cleansing, astringent, and moisturizing effects are also obtained. Examples of the sugars include hydrolyzed and hydrogenated starch. By containing the sugars, a moisturizing effect and an anti-inflammatory effect are improved. Examples of the proteins include collagen. By containing the proteins, a vehicle for calcium phosphate is provided to promote the delivery into a dermic layer. Examples of the amino acids include glutamic acid, alginic acid and sodium salts thereof. By containing the amino acids, collagen production can be enhanced. Examples of the water-soluble vitamins include magnesium ascorbyl phosphate. By containing the water-soluble vitamins, collagen production is enhanced and a synergistic effect with calcium phosphate is expected. Examples of the fat-soluble vitamins include vitamin A. By containing the fat-soluble vitamins, production of mucosaccharides is promoted to give moisture on skin. Examples of the lipids include ceramides and phospholipids. By containing the lipids, a moisturizing effect is obtained. Examples of the mucosaccharides include hyaluronic acid. By containing the mucosaccharides, a moisturizing effect is obtained. Examples of the surface-active agent include polyethylene glycol and stearic acid derivatives. By containing the surface-active agent, dispersability of fat-soluble component such as fine particles and vitamin A is improved.

The calcium phosphate fine particles according to the invention are useful to be added to a cosmetic. The type of cosmetics is not specifically limited, and specific examples thereof include facial washing agent, skin lotion, beauty essence, ointment, cream, emulsion, lotion, facial mask, rouge and bath additive. The addition amount of the calcium phosphate fine particles in the cosmetic according to the most preferred embodiment of the invention can be suitably adjusted according to the type of cosmetic or physiological activity of an extract, etc. It is preferably 0.01-30 mass-%, more preferably 0.1-15 mass-%, and still more preferably 0.1-10 mass-%. The addition amount of the alcohols is not specifically limited, but it is preferably 0.01-70 mass-%, more preferably 0.01-50 mass-%, and still more preferably 0.01-30 mass-% . The addition amount of the sugars is not specifically limited, but it is preferably 0.0170 mass-%, more preferably 0.01-50 mass-%, and still more preferably 0.01-30 mass-%. The addition amount of the proteins is not specifically limited, but it is preferably 0.01-30 mass-%, more preferably 0.01-20 mass-%, and still more preferably 0.01-10 mass-%. The addition amount of the amino acids is not specifically limited, but it is preferably 0.01-30 mass-%, more preferably 0.01-20 mass-%, and still more preferably 0.01-10 mass-%. The addition amount of the water-soluble vitamins is not specifically limited, but it is preferably 0.001-30 mass-%, more preferably 0.001-20 mass-%, and still more preferably 0.001-10 mass-%. The addition amount of the fat-soluble vitamins is not specifically limited, but it is preferably 0.001-30 mass-%, more preferably 0.001-20 mass-%, and still more preferably 0.001-10 mass-%. The addition amount of the lipids is not specifically limited, but it is preferably 0.001-30 mass-%, more preferably 0.001-20 mass-%, and still more preferably 0.001-10 mass-%. The addition amount of the mucosaccharides is not specifically limited, but it is preferably 0.01-70 mass-%, more preferably 0.01-50 mass-%, and still more preferably 0.01-30 mass-%. The addition amount of the surface-active agents is not specifically limited, but it is preferably 0.001-30 mass-%, more preferably 0.001-20 mass-%, and still more preferably 0.001-10 mass-%.

The cosmetic according to the most preferred embodiment of the invention may use various principle components and aiding components, and other optional aiding components that are generally used for production of cosmetics, as long as the activity of enhancing collagen production by calcium phosphate fine particles is not impeded by them. According to the cosmetic according to the most preferred embodiment of the invention, examples of the components that can be used, along with the calcium phosphate fine particles, as a constitutional component of a cosmetic include additives that are well known in the art. Specific examples include an astringent, a bactericidal · anti-microbial agent, a whitening agent, a UV absorbing agent, a moisturizing agent, a cell revitalizing agent, an anti-inflammatory · anti-allergic agent and an anti-oxidizing active oxygen scavenging agent. Further, when a cosmetic is to be produced, choice of other raw production materials is hardly limited. Any common base materials or auxiliaries, which include, for example, fats and oils, waxes, hydrocarbons, fatty acids, alcohols, esters, surface active agent and fragrances, can be used.

### <<Production method>>

Next, the method for production of the calcium phosphate fine particles according to the most preferred embodiment of the invention is explained. The calcium phosphate fine particles may be artificially synthesized by a known production method such as a wet method, a dry method, a hydrolysis method, and a hydrothermal method, or it may be originated from natural product such as bone and teeth. Further, calcium phosphate particles having a large particle diameter are prepared and then pulverized by a method well known in the field.

The calcium phosphate fine particles are preferably obtained by sintering amorphous calcium phosphate. The lower limit for sintering temperature is 100°C or more. When the sintering temperature is less than 100°C, sufficient sintering may not be obtained. Meanwhile, the upper limit for sintering temperature is ≤ 1800°C, more preferably ≤ 1250°C, and still more preferably ≤ 1200°C. When the sintering temperature is > 1800°C, calcium phosphate may be decomposed. Thus, by having the sintering temperature within the range above, calcium phosphate hardly soluble in a living body (i.e. highly crystalline) can be produced. The sintering time is not specifically limited, and it can be suitably selected. Further, although there can be a case in which particles are fused to each other by sintering, it is possible to use the sintered particles after pulverizing them.

The calcium phosphate fine particles according to the preferred embodiment of the invention are particularly preferably produced by the method described below. Specifically, the method for producing the calcium phosphate fine particles according to the preferred embodiment of the invention is suitably a method including dispersion calcination (sintering) which consists of, at least, a mixing process and a sintering process. As the particle diameter of the primary particles is faithfully reflected on the fine particles obtained by dispersion calcination, particles having the average particle diameter within the range described above can be easily produced. Further, the production method according to the preferred embodiment of the invention may contain a process of producing primary particles and a removal process. These processes are performed, for example, in an order of a process for producing primary particles, a mixing process, a calcination process, and a removal process.

### (Process for producing primary particles)

The process for producing primary particles is not specifically limited, as long as it is a process enabling the production of calcium phosphate fine particles. It can be suitably selected and used depending on raw materials of highly crystalline calcium phosphate fine particles. For example, when phosphoric acid is added dropwise to a slurry of calcium hydroxide at room temperature, particles of calcium phosphate (CaP) precipitate.

A method for production of primary particle group having homogeneous particle diameter (i.e. narrow particle size distribution) and fine particle size (i.e. nanometer size) as in the calcium phosphate fine particles according to the preferred embodiment of the invention is not specifically limited, and a method disclosed in JP-A-2002-137910 can be used, for example. Specifically, a calcium solution and a phosphoric acid solution are solubilized and mixed in an emulsion phase containing surface active agent/water/oil and are allowed to react at a temperature above the cloud point of the surface-active agent, and therefore calcium phosphate (hydroxyapatite) fine particles (primary particles) can be synthesized. In addition, by modifying the functional groups and hydrophilicity/hydrophobicity ratio in the surface-active agent, size of the calcium phosphate fine particles can be controlled.

Simple explanation of the principles for producing the calcium phosphate fine particles is as follows. According to a method in which a calcium solution and a phosphoric acid solution are solubilized and mixed in an emulsion phase containing surface active agent/water/oil and are allowed to react for the synthesis of calcium phosphate fine particles, nucleus of calcium phosphate and particles grow in micelles of a surface-active agent. At that time, by modifying the reaction temperature (when the surface-active agent is a non-ionic surface-active agent, it is above the cloud point of the surface-active agent), thermodynamic stability of the micelles can be controlled. In other words, increasing the reaction temperature represents reducing the force for forming micelles of the surface-active agent. Accordingly, it is believed that a driving force for particle growth of the calcium phosphate, which has been limited within the boundary of the micelles, becomes higher than the driving force to maintain the boundary of the micelles. Thus, by utilizing this mechanism, it is possible to control the shape of the particles.

When micelles of a surface-active agent are produced, functional groups (hydrophilic moiety) of the surface-active agent and hydrophilicity/hydrophobicity ratio in the molecule are important. According to a difference in them, stability and cloud point of the micelles may also vary. The cloud point of a surface-active agent varies depending on the type of surface-active agent. As such, by appropriately modifying the type of surface-active agent, functional groups of the surface-active agent and hydrophilicity/hydrophobicity ratio can be varied, and therefore it is possible to control the size of the calcium phosphate fine particles.

The type of surface-active agent that is used for the method described above is not specifically limited, and it may be appropriately selected from other kinds of known anionic, cationic, amphoteric ionic, and non-ionic surface-active agents disclosed in JP-A-5-17111 and used. Among them, the non-ionic surface-active agent has a cloud point of a surface-active agent, and therefore shape of the crystal can be easily controlled based on the mechanism described above. More specific examples of the non-ionic surface-active agent that can be used include polyoxyethylene alkyl ether, polyoxyethylene aryl ether, polyoxyethylene alkylaryl ether, polyoxyethylene derivatives, oxyethylene · oxypropylene block copolymer, sorbitan fatty acid ester, polyoxyethylene sorbitan fatty acid ester, polyoxyethylene sorbitol fatty acid ester, glycerin fatty acid ester, polyoxyethylene fatty acid ester and polyoxyethylene alkylamine. Examples of the cationic surface-active agent that can be used include quaternary ammonium salts such as stearylamine hydrochloride, lauryl trimethyl ammonium chloride, and alkyl benzene dimethyl ammonium chloride. Examples of the anionic surface-active agent that can be used include higher alcohol sulfuric acid ester salts such as sodium lauryl alcohol sulfate and sodium oleyl alcohol sulfate, alkyl sulfuric acid salts such as sodium lauryl sulfate and ammonium lauryl sulfate, and alkylaryl sulfonic acid salts such as sodium dodecyl benzene sulfonate and sodium dodecyl naphthalene sulfonate. Examples of the amphoteric surface-active agent that can be used include alkyl betaine type, alkyl amide betaine type and amine oxide type. The surface-active agent may be used either singly or in combination of two or more. Among them, from the viewpoint of cloud point and solubility, it is particularly preferable to use pentaethylene glycol dodecyl ether.

Examples of the oil phase that can be used for the method described above include hydrocarbons such as toluene, xylene, hexane, dodecane, and cyclohexane, halogenated hydrocarbons such as chlorobenzene and chloroform, ethers such as diethyl ether, alcohols such as butanol and ketones such as methyl isobutyl ketone and cyclohexanone. To have low solubility in water depending on the type of surface-active agent used and to dissolve any one of the surface-active agents, the solvent may be selected either singly or in combination of two or more. Among them, from the viewpoint of solubility in water and solubility of a surface-active agent, it is particularly preferable to use dodecane. The reaction temperature, reaction time, and addition amount of reacting materials can be selected according to an optimum condition determined by the composition of the primary particles, and used. However, as the reaction is an aqueous solution reaction, the upper limit of the reaction temperature is preferably a temperature at which the solution does not boil. Preferably, it is ≤ 90°C.

The present process may also include a process of washing the produced primary particles with e.g. water and a process of recovering the primary particles by e.g. centrifuge or filtration.

### (Mixing process)

The mixing process is a process for mixing the primary particles and a fusion preventive agent. By incorporating in advance, a fusion preventive agent between the particles of the primary particle group that are obtained by the process for producing primary particles, fusion among the primary particles during the following sintering process can be prevented. In addition, the mixture of the primary particles obtained by the mixing process and a fusion preventive agent is referred to as "mixed particles."

As used herein, the "fusion preventive agent" is not specifically limited as long as it can prevent the fusion among primary particles. However, an agent which is not volatile at the sintering temperature of the following sintering process is preferable. As being non-volatile at sintering temperature condition, it is not lost among the primary particles during sintering process and the fusion among the primary particles is surely prevented. However, it is not necessary to have 100% non-volatility at the sintering temperature. Instead, non-volatility allowing presence of ≥ 10% within the primary particles after completion of the sintering process is acceptable. Further, the fusion preventive agent may be chemically decomposed by heat after completion of the sintering process . In other words, as long as it remains after completion of the sintering process, it is not necessary to be the same substance (compound) before and after the sintering process.

It is preferable that the fusion preventive agent is soluble in a solvent, in particular an aqueous solvent. When a fusion preventive agent which can be dissolved in a solvent is used as a fusion preventive agent, the fusion preventive agent (e.g. calcium carbonate) can be removed only by suspending the calcium phosphate fine particles in an aqueous solvent such as pure water. In particular, a fusion preventive agent which can be dissolved in an aqueous solvent does not need any organic solvent for removing the fusion preventive agent. Thus, facilities and treatment of organic solvent waste liquid, that are resulted from the use of an organic solvent during removal process, are unnecessary. Therefore, it can be said that the fusion preventive agent can be removed more conveniently from the calcium phosphate fine particles. Examples of the solvent include, although not being specifically limited, an aqueous solvent such as water, ethanol and methanol, and examples of the organic solvent include acetone and toluene.

Further, to increase the solubility of a fusion preventive agent in water, the aqueous solvent may contain a chelate compound such as oxalate, ethylene diamine, bipyridine, and ethylene diamine tetraacetate. Still further, to increase the solubility of a fusion preventive agent in water, the aqueous solvent may contain an electrolyte ion such as sodium chloride, ammonium nitrate, and potassium carbonate.

Higher solubility of the fusion preventive agent in solvent is preferable because the removal efficiency increases in accordance with the solubility. The preferred solubility is ≥ 0.01 g when the solubility represents the amount (g) of solute in 100 g of a solvent. More preferably, it is ≥ 1 g, and most preferably ≥ 10 g.

Specific examples of the fusion preventive agent include calcium salts (or complexes) such as calcium chloride, calcium oxide, calcium sulfate, calcium nitrate, calcium carbonate, calcium hydroxide, calcium acetate and calcium citrate, potassium salts such as potassium chloride, potassium oxide, potassium sulfate, potassium nitrate, potassium carbonate, potassium hydroxide, potassium phosphate, and sodium salts such as sodium chloride, sodium oxide, sodium sulfate, sodium nitrate, sodium carbonate, sodium hydroxide, and sodium phosphate.

Further, the method of mixing the primary particles with a fusion preventive agent during the mixing process is not specifically limited. A method of mixing the solid primary particles with a solid fusion preventive agent and further mixing them using a blender can be used. A method of dispersing the primary particles in a solution of a fusion preventive agent can be carried out. However, since homogenous mixing of a solid with other solid is rather difficult to achieve, it can be said the latter is a preferable method to insert surely and evenly the fusion preventive agent between the primary particles. When the latter method is adopted, it is preferable that a solution of fusion preventive agent in which the primary particles are dispersed is prepared in a dried state, because a homogenous mixed state of the primary particles and the fusion preventive agent can be maintained for a long period of time by it. According to the Examples described below, 0.5 g of the hydroxyapatite (HAp) primary particles is dispersed in a saturated aqueous solution of calcium carbonate and dried at 80°C to obtain the mixed particles.

Further, the mixing process may be a process for adding metal salts (alkali metal salts and/or alkali earth metal salts and/or transition metal salts) by mixing a solution containing a polymer compound having any one of a carboxyl group, a sulfuric acid group, a sulfonic acid group, a phosphoric acid group, a phosphonic acid group, and an amino group in a side chain, or salts thereof with the primary particles. By having this process, the polymer compound is adsorbed on the surface of calcium phosphate {hydroxyapatite (HAp)} to completely block a contact among calcium phosphate {hydroxyapatite (HAp)} during a process of mixing a fusion preventive agent. Further, by adding calcium salts after that, it is possible to ensure precipitation of a fusion preventive agent on the surface of calcium phosphate {hydroxyapatite (HAp) } . Herein below, a polymer compound having any one of a carboxyl group, a sulfuric acid group, a sulfonic acid group, a phosphoric acid group, a phosphonic acid group, and an amino group in a side chain, or salts thereof is simply referred to as a "polymer compound."

The polymer compound is not specifically limited as long as it is a compound having any one of a carboxyl group, a sulfuric acid group, a sulfonic acid group, a phosphoric acid group, a phosphonic acid group, and an amino group in a side chain, or salts thereof. Examples of a polymer compound having a carboxyl group in a side chain include polyacrylic acid, polymethacrylic acid, sodium polyacrylate, sodium polymethacrylate, carboxymethyl cellulose and a styrene-maleic anhydride copolymer. Examples of a polymer compound having a sulfuric acid group in a side chain include polyacrylic acid alkyl sulfate, polymethacrylic acid alkyl sulfate and polystyrene sulfuric acid. Examples of a polymer compound having a sulfonic acid group in a side chain include polyacrylic acid alkyl sulfonate, polymethacrylic acid alkyl sulfonate and polystyrene sulfonic acid. Examples of a polymer compound having a phosphoric acid group in a side chain include polyacrylic acid alkyl phosphate, polymethacrylic acid alkyl phosphate, polystyrene phosphoric acid and polyacryloyl amino methyl phosphoric acid. Examples of a polymer compound having a phosphonic acid group in a side chain include polyacrylic acid alkyl phosphonate, polymethacrylic acid alkyl phosphonate, polystyrene phosphonic acid, poly acryloyl amino methyl phosphonic acid and polyvinyl alkyl phosphonic acid. Examples of a polymer compound having an amino group in a side chain include polyacrylamide, polyvinyl amine, polymethacrylic acid aminoalkyl ester, polyaminostyrene, a polypeptide and a protein. For the mixing process, any one kind of the polymer compounds above can be used. However, a mixture containing plural kinds of the polymer compound can be also used.

Molecular weight of the polymer compound is not specifically limited. Preferably it is 100-1,000,000 g/mol, more preferably 500-500,000 g/mol, and most preferably 1, 000-300, 000 g/mol. When it is less than the range, introduction ratio to the primary particles is low so that the ratio of inhibiting contact among the primary particles is lowered. On the other hand, when the ratio is above the range, it is undesirable that operability is deteriorated as follows: the solubility of the polymer compound is reduced and the viscosity of the solution containing the polymer compound is increased.

The solution containing the polymer compound is preferably an aqueous solution, because the sintered particles of calcium phosphate {hydroxyapatite (HAp) } are dissolved under a strongly acidic condition. Further, pH of the aqueous solution containing the polymer compound is not specifically limited, as long as it has pH of 5-14 and HAp particles are insoluble therein. pH of the aqueous solution containing the polymer compound can be adjusted with an aqueous solution of e.g. ammonia, sodium hydroxide or potassium hydroxide after dissolving the polymer compound in e.g. pure water or ion-exchange water.

Concentration of the polymer compound contained in the aqueous solution is preferably 0.001-50% w/v, more preferably 0.005-30% w/v, and still more preferably 0.01-10% w/v. When it is less than the range, introduction amount to the primary particles is low so that the ratio of inhibiting contact among the primary particles is lowered. On the other hand, when the ratio is above the range, it is undesirable that operability is deteriorated as follows: the solubility of the polymer compound is reduced and the viscosity of the solution containing the polymer compound is increased.

According to the mixing process of the invention, the solution containing the polymer compound and the primary particles are admixed with each other. The mixing can be carried out, for example, by adding the primary particles to the solution and dispersing the primary particles via stirring. According to the treatment, surface of the primary particles are adsorbed with the polymer compound according to the method for producing calcium phosphate of the invention, and as a result, any one of a carboxyl group, a sulfuric acid group, a sulfonic acid group, a phosphoric acid group, a phosphonic acid group, an amino group, or salts thereof can be added to the surface of the primary particles. In this case, a carboxyl group, a sulfuric acid group, a sulfonic acid group, a phosphoric acid group, a phosphonic acid group, or an amino group is present in an ion state in the solution.

Next, by further adding metal salts (alkali metal salts and/or alkali earth metal salts and/or transition metal salts) to a solution obtained by mixing a solution containing the polymer compound with the primary particles, the metal ions (alkali metal ions and/or alkali earth metal ions and/or transition metal ions) bind to a carboxylic acid ion, a sulfuric acid ion, a sulfonic acid ion, a phosphoric acid ion, a phosphonic acid ion, or an amino ion that are present on the surface of the primary particles to yield a carboxylate, a sulfate, a sulfonate, a phosphate, a phosphonate, and an amino acid salt on the surface of the primary particles. A carboxylate, a sulfate, a sulfonate, a phosphate, a phosphonate, or an amino acid salt of the metal (alkali metal and/or alkali earth metal and/or transition metal) function as the fusion preventive agent explained above. Thus, the primary particles having a surface on which a carboxylate, a sulfate, a sulfonate, a phosphate, a phosphonate, or an amino acid salt of the metal (alkali metal and/or alkali earth metal and/or transition metal) is formed are so-called "mixed particles." In addition, as the carboxylate, sulfate, sulfonate, phosphate, phosphonate, and amino acid salt of the metal (alkali metal and/or alkali earth metal and/or transition metal) precipitate, the precipitates are recovered, dried, and can be subjected to the sintering process that is explained below. The drying can be carried out according to a method performed under heating (preferably at 0-200°C, more preferably 20-150°C, and most preferably 40-120°C) and reduced pressure (preferably between 1 × 10⁵ Pa and 1 × 10⁻⁵ Pa, more preferably between 1 × 10³ Pa and 1 × 10⁻³ Pa, and most preferably between 1 × 10² Pa and 1 × 10⁻² Pa). In addition, although the drying under reduced pressure is preferable as the drying temperature can be lowered, it can be also carried out under atmospheric pressure.

Examples of the alkali metal salts that can be used include, although not specifically limited, sodium chloride, sodium hypochlorite, sodium chlorite, sodium bromide, sodium iodide, sodium iodate, sodium oxide, sodium peroxide, sodium sulfate, sodium thiosulfate, sodium selenate, sodium nitrite, sodium nitrate, sodium phosphide, sodium carbonate, sodium hydroxide, potassium chloride, potassium hypochlorite, potassium chlorite, potassium bromide, potassium iodide, potassium iodate, potassium oxide, potassium peroxide, potassium sulfate, potassium thiosulfate, potassium selenate, potassium nitrite, potassium nitrate, potassium phosphide, potassium carbonate and potassium hydroxide.

Further, examples of the alkali earth metal salts that can be used include magnesium chloride, magnesium hypochlorite, magnesium chlorite, magnesium bromide, magnesium iodide, magnesium iodate, magnesium oxide, magnesium peroxide, magnesium sulfate, magnesium thiosulfate, magnesium selenate, magnesium nitrite, magnesium nitrate, magnesium phosphide, magnesium carbonate, magnesium hydroxide, calcium chloride, calcium hypochlorite, calcium chlorite, calcium bromide, calcium iodide, calcium iodate, calcium oxide, calcium peroxide, calcium sulfate, calcium thiosulfate, calcium selenate, calcium nitrite, calcium nitrate, calcium phosphide, calcium carbonate and calcium hydroxide.

Further, examples of the transition metal salts that can be used include zinc chloride, zinc hypochlorite, zinc chlorite, zinc bromide, zinc iodide, zinc iodate, zinc oxide, zinc peroxide, zinc sulfate, zinc thiosulfate, zinc selenate, zinc nitrite, zinc nitrate, zinc phosphide, zinc carbonate, zinc hydroxide, iron chloride, iron hypochlorite, iron chlorite, iron bromide, iron iodide, iron iodate, iron oxide, iron peroxide, iron sulfate, iron thiosulfate, iron selenate, iron nitrite, iron nitrate, iron phosphide, iron carbonate, and iron hydroxide. A nickel compound can be also used.

The metal salts (alkali metal salts and/or alkali earth metal salts and/or transition metal salts) that are added to a solution obtained by mixing a solution containing the polymer compound and the primary particles may be either a single type or a mixture of two or more types. Further, the metal salts (alkali metal salts and/or alkali earth metal salts and/or transition metal salts) in a solid state may be used. However, from the viewpoint of e.g. even addition and control of the addition concentration, it is preferably added as an aqueous solution. Further, the addition amount (i.e. concentration) of the metal salts (alkali metal salts and/or alkali earth metal salts and/or transition metal salts) is not specifically limited as long as it allows the binding of a carboxylic acid ion, a sulfuric acid ion, a sulfonic acid ion, a phosphoric acid ion, a phosphonic acid ion, or an amino ion to form a carboxylate salt, a sulfate salt, a sulfonate salt, a phosphate salt, a phosphonate salt or an amino acid salt of the metal (alkali metal and/or alkali earth metal and/or transition metal). It can be selected after appropriate determination.

Further, when the carboxylate, sulfate, sulfonate, phosphate, phosphonate, or amino acid salt of the metal (alkali metal and/or alkali earth metal and/or transition metal) formed on the surface of the primary particles according to the process described above is subjected to thermal decomposition during the sintering process described below, metal (alkali metal and/or alkali earth metal and/or transition metal) oxides are generated. For example, when calcium polyacrylate is formed on the surface of primary particles, calcium oxide is generated by sintering process. Further, since the metal oxides (alkali metal oxides and/or alkali earth metal oxides (e.g. calcium oxide) and/or transition metal oxides) are water soluble, they can easily removed by the removal process described below.

Sodium polyacrylate is soluble in water, and therefore, as a fusion preventive agent, it can be used as it is in the mixing process. However, calcium polyacrylate is insoluble in water. Thus, it is preferable that only polyacrylic acid is adsorbed on the surface of the primary particles and then, for example, calcium salts are added to precipitate the calcium polyacrylate on the surface of the primary particles. Further, when the primary particles are calcined at high temperature (about 300°C or higher), the polymer compound is decomposed. Thus, it can be said that, for the compound to function as a fusion preventive agent even after calcination, it is preferable that the metal salts of the polymer compound are first precipitated on the surface of the primary particles. However, when the calcination (heat treatment) of the primary particles is carried out at the temperature at which the polymer compound does not decompose (i.e. not softens), it is unnecessary to precipitate particularly the metal salts of the polymer compound on the surface of the primary particles.

### (Sintering process)

The sintering process is a process of exposing the mixed particles obtained from the mixing process to sintering temperature and obtaining the primary particles contained the mixed particles as highly crystalline phosphate fine particles (sintered body particles). As the fusion preventive agent is incorporated between the particles of the primary particles, the fusion among the primary particles can be prevented even when they are exposed at high temperature during sintering process.

The sintering temperature for the sintering process can be appropriately selected to achieve the hardness of the highly crystalline calcium phosphate fine particles at desired level. For example, it is within the range of 100-1, 800°C, more preferably 150-1,500°C, and most preferably 200-1,200°C. Further, the sintering time can be appropriately selected in view of the desired hardness of the highly crystalline calcium phosphate fine particles. In the Examples described below, the sintering is carried out at 800°C for 1 hour.

Further, the device used for the sintering process is not specifically limited. It can be used after appropriately selecting a commercially available sintering furnace depending on e.g. the production scale and the production condition.

### (Removal process)

The removal process is a process for removing the fusion preventive agent that are scattered between the highly crystalline calcium phosphate fine particles obtained from the sintering process.

Means and method for removal can be appropriately selected depending on the fusion preventive agent used in the mixing process. For example, when a fusion preventive agent having solvent solubility is used, only the fusion preventive agent can be dissolved and removed by using a solvent which does not dissolve calcium phosphate fine particles (non-dissolving) but dissolves the fusion preventive agent (dissolving). The solvent that to be used is not specifically limited, as long as it satisfies the requirements above, and it can be an aqueous solvent or an organic solvent. Examples of the aqueous solvent include water, ethanol and methanol. Examples of the organic solvent include acetone and toluene.

Further, to increase the solubility of a fusion preventive agent in water, the aqueous solvent may contain a chelate compound such as oxalate, ethylene diamine, bipyridine, and ethylene diamine tetraacetate. Still further, to increase the solubility of a fusion preventive agent in water, the aqueous solvent may contain an electrolyte ion such as sodium chloride, ammonium nitrate, and potassium carbonate.

However, from the viewpoint that, for example, during the removal process, facilities corresponding to the use of an organic solvent and treatment of organic solvent waste liquid are unnecessary, safety for the production operation is high, and the environmental risk is low, the solvent to be used is preferably an aqueous solvent.

Further, in case of particles of a sintered body of highly crystalline calcium phosphate {hydroxyapatite (HAp) }, particles of a sintered body of highly crystalline calcium phosphate {hydroxyapatite (HAp) } are dissolved under the condition with pH of ≤ 4.0. Thus, it is preferable to carry out the removal process at pH of 4.0-12.0.

Meanwhile, when the fusion preventive agent is removed by using a solvent, it is possible that calcium phosphate containing the fusion preventive agent obtained from the sintering process is suspended in a solvent, and then only the calcium phosphate particles are recovered by centrifuge. According to the method for producing calcium phosphate according to the most preferred embodiment of the invention, this step is not limited to a single operation. Rather, it may be performed two or more times. It can be said that the removal ratio of the fusion preventive agent from calcium phosphate is further increased by performing the above step two or more times. However, from the viewpoint that the production process is complicated, production cost is increased, and recovery ratio of calcium phosphate is lowered, it is not desirable to perform the step more than it is required. Thus, the number of repeating the step is appropriately selected in view of the target removal ratio of the fusion preventive agent.

The process may further contain a sizing process to obtain even particle diameter.

In addition to the above method of using a solvent to remove a fusion preventive agent, the fusion preventive agent can be removed by using a magnetic fusion preventive agent and a magnet. More specifically, a group of calcium phosphate particles (crude calcium phosphate particles) containing the fusion preventive agent obtained from the sintering process are suspended and dispersed in a suitable solvent (e.g. water), magnetic force is applied to the suspension to adsorb only the fusion preventive agent on the magnet, and non-adsorbed calcium particles are selectively recovered. In addition, it is also possible to perform a method including smashing the crude calcium phosphate particles without specifically suspending them in a solvent and separating the fusion preventive agent by using a magnet. However, it can be said that the suspension is favorable in that the calcium phosphate particles and the fusion preventive agent can be easily separated from each other and the removal ratio of the fusion preventive agent is high. Further, it is preferable that the calcium phosphate particles to which this method can be applied is a non-magnetic body or a weakly-magnetic body.

### <<Properties>>

In the calcium phosphate particles that are produced according to the method for producing calcium phosphate particles of the most preferred embodiment of the invention, fusion among the primary particles is inhibited by an action of the fusion preventive agent, and therefore majority of the particles maintain a particle state. Therefore, when the highly crystalline calcium phosphate particles are suspended in a solvent, it can be dispersed as a particle mass (monocrystalline primary particles) in which the primary particles consisting of highly crystalline calcium phosphate particles, wherein majority of them consist of monocrystals, or the primary particles consisting of the monocrystals are grouped together via an ionic interaction.

The calcium phosphate particles according to the most preferred embodiment of the invention are a particle mass (monocrystalline primary particles) in which the primary particles mostly consisting of monocrystals, or the primary particles consisting of the monocrystals are grouped together via an ionic interaction, and they have good dispersability in a solvent and, as having no secondary particles, have a high surface area.

Herein, as a method for evaluating whether or not the calcium phosphate fine particles are present as primary particles, when the particles diameter measured by an observation with an electron microscope is almost the same as the particle diameter measured by dynamic light scattering in a suspension state in a solvent, it can be determined that the majority of the calcium phosphate particles according to the most preferred embodiment of the invention are in a primary particle state. On the other hand, when the particle diameter measured by dynamic light scattering in a suspension state in a solvent is larger than the particles diameter measured by an observation with an electron microscope, it can be determined that the fusion among the primary particles occurs to form secondary particles.

Further, the solvent for dispersing the calcium phosphate particles according to the most preferred embodiment of the invention is not specifically limited, as long as it does not dissolve the calcium phosphate particles. Examples thereof include water, alcohols such as methanol and ethanol, ketones such as acetone, methyl ethyl ketone, methyl isobutyl ketone, and cyclohexanone, amides such as N,N-dimethyl formamide, sulfoxides such as dimethyl sulfoxide, hydrocarbons such as toluene, xylene, hexane, dodecane, and cyclohexane, halogenated hydrocarbons such as chlorobenzene and chloroform, and ethers such as diethyl ether and dioxane. These solvents may be used either singly or in combination of two or more, depending on the purpose of use.

By obtaining the ratio of the particles which have a particle diameter almost the same as the particle diameter of the primary particles obtained by using an microscope based on the particle diameter distribution obtained by dynamic scattering method, the ratio of a particle mass (monocrystalline primary particles) in which the primary particles consisting of monocrystals, or the primary particles consisting of the monocrystals are grouped together via an ionic interaction can be calculated.

Further, although it may vary according to, for example, a raw material for calcium phosphate, the type of fusion active agent, and sintering condition, according to the method for producing highly crystalline calcium phosphate particles according to the most preferred embodiment of the invention, at least 50%, more preferably at least 60%, and most preferably at least 70%, are present as monocrystalline primary particles.

### (Administration method and dosage)

Examples of a means for delivery of the collagen production enhancer according to the most preferred embodiment of the invention to an administration site include direct coating on an application area, transdermal absorption, subcutaneous injection and surgical procedures.

The collagen production enhancer according to the most preferred embodiment of the invention can exhibit its effect by using it as a cosmetic twice a day and 150 mg per use (provided that, 1.5 mg of calcium phosphate is included therein).

### EXAMPLES

### Production Example 1 (production of calcium phosphate fine particles)

### (Process for production of primary particles)

Dodecane [CH₃(CH₂)₁₀CH₃] and pentaethylene glycol dodecyl ether [CH₃(CH₂)₁₀CH₂O(CH₂CH₂O)₄CH₂CH₂OH] having cloud point of 31°C were used as a continuous oil phase and a non-ionic surface active agent, respectively. At room temperature, a continuous oil phase (40 ml) containing 0.5 g of the non-ionic surface active agent was produced. Thereafter, 10 ml of 2.5 mol/l aqueous dispersion solution of calcium hydroxide [Ca(OH)₂] was added to the continuous oil phase produced above to prepare a water-in-oil type solution (W/O solution) . 10 ml of 1.5 mol/l potassium dihydrogen phosphate solution [(KH₂PO₄)] was added to the W/O solution under stirring. The reaction was allowed to progress for 24 hours at room temperature under stirring. Consequently, the reaction product obtained was isolated and washed by centrifuge to obtain primary particle group of hydroxyapatite (HAp).

### (Mixing process)

To 100 ml aqueous solution of pH 12.0 containing 1.0 g of sodium polyacrylate (manufactured by ALDRICH Corporation, weight average molecular weight of 15,000 g/mol), 1.0 g of the primary particle group of hydroxyapatite (HAp) was dispersed so that surface of the particles is adsorbed with sodium polyacrylate. pH of the aqueous solution was measured by D-24SE (trade name), a pH meter manufactured by HORIBA, Ltd.

Next, 100 ml of 0.12 mol/l aqueous solution of calcium nitrate [Ca(NO₃)₂] was added to the dispersion obtained above so that the calcium polyacrylate is precipitated on the surface of the primary particles. The calcium polyacrylate corresponds to a fusion preventive agent. The precipitates generated were recovered and dried at 80°C under reduced pressure (about 0.1 Pa) to obtain mixed particles.

### (Sintering process)

The mixed particles were placed in a crucible and sintered for 1 hour at sintering temperature of 800°C. Accordingly, calcium polyacrylate was decomposed by heat and turned into calcium oxide [CaO] . The remaining ratio of calcium oxide [CaO] after completion of the sintering process was 25% or more.

### (Removal process)

To increase the solubility of a fusion preventive agent in water, 50 mmol/l aqueous solution of ammonium nitrate [NH₄NO₃] was prepared. Thereafter, the sintered body obtained from the above process was suspended in 500 ml of the aqueous solution prepared, and separated and washed by centrifuge. By further suspending in distilled water, separation and washing was carried out in a similar manner by centrifuge to remove the fusion preventive agent and ammonium nitrate. As a result, fine particles of highly crystalline hydroxyapatite (HAp) were dissolved. Detailed information of fine particles of hydroxyapatite obtained by the above steps is summarized below.

Half width of XRD: 0.2 (d = 2.814)
Shape: spherical
Average particle diameter (under electron microscope) : 28 nm
Variation coefficient: 14%

### Production Example 2 (non-sintered) (reference)

Only the process for producing primary particles was carried out under the same condition as the Production example 1 except that the reaction temperature for the process for producing primary particles is changed to 30°C. Without performing the subsequent processes such as mixing process and sintering process, fine particles of non-sintered hydroxyapatite of Production Example 2 were obtained. Detailed information of fine particles of hydroxyapatite obtained is summarized below.

Half width of XRD: 0.8 (d = 2.814)
Shape: particle shape
Average particle diameter (under electron microscope) : 42 nm
Variation coefficient: 17%

### <Test for determining activity of fibroblast to enhance type I collagen production>

Fibroblasts of a normal human were cultured for 24 hours in 0.5% FBS-DMEM culture medium containing the test sample, and the amount of collagen in the culture medium was quantified by ELISA. The test was carried out by repeating six times for each condition (n = 6). As a positive control (P.C.), magnesium ascorbyl phosphate (VCPMg) was used. The test samples employed were the calcined hydroxyapatite that is produced in the Production Example 1 and the non-calcined hydroxyapatite that is used in the Production Example 2 (reference). The results are given in Table 1. As used herein, the term "mean" in Table 1 indicates a measured collagen amount obtained by measurement (i.e. mean value), the term 'SD" represents standard deviation, and the term "p (t-test)" represents the p value obtained by "t-test" {in general, when it is the same or less than 0.05 (0.01 in strict sense), it indicates that there is a difference, while it is greater than 0.05, it indicates that there is almost no difference}.

**[Table 1]**

| | | Activity of enhancing collagen production of fibroblast | | | | **(n=6)** | | | |
|---|---|---|---|---|---|---|---|---|---|
| Sample | Cone. (µg/mL) | Collagen (µg / mg protein) | | | | Protein (µg / well) | | | |
| | | Mean | ± | SD | ²⁾ p (t-test) | Mean | ± | SD | ²⁾ p (t-test) |
| Preparation Example 1 Hydroxy apatite (calcined) | 0 | 9.84 | ± | 0.40 | 1.000 | 6.80 | ± | 0.38 | 1.000 |
| | ¹⁾ P.C. | 24.28 | ± | 1.06 | 0.000 | 6.99 | ± | 0.56 | 0.516 |
| | 0.80 | 14.99 | ± | 1.43 | 0.000 | 6.65 | ± | 0.55 | 0.603 |
| | 1.60 | 16.69 | ± | 0.75 | 0.000 | 6.67 | ± | 0.23 | 0.484 |
| | 3.10 | 16.66 | ± | 1.07 | 0.000 | 6.62 | ± | 0.42 | 0.443 |
| | 6.30 | 18.06 | ± | 1.12 | 0.000 | 6.84 | ± | 0.31 | 0.846 |
| | 12.50 | 16.94 | ± | 0.53 | 0.000 | 6.60 | ± | 0.25 | 0.310 |
| | 25.00 | 16.93 | ± | 1.30 | 0.000 | 6.63 | ± | 0.26 | 0.379 |
| | 50.00 | 16.17 | ± | 0.96 | 0.000 | 7.03 | ± | 0.26 | 0.256 |
| | 100.00 | 14.29 | ± | 0.38 | 0.000 | 7.08 | ± | 0.34 | 0.209 |
| Preparation Example 2 Hydroxy apatite (non-calcined) | 0 | 9.16 | ± | 0.61 | 1.000 | 6.34 | ± | 0.36 | 1.000 |
| | ¹⁾P.C. | 19.75 | ± | 1.11 | 0.000 | 6.36 | ± | 0.21 | 0.878 |
| | 0.80 | 9.51 | ± | 0.62 | 0.347 | 6.22 | ± | 0.17 | 0.473 |
| | 1.60 | 9.79 | ± | 0.64 | 0.113 | 6.27 | ± | 0.16 | 0.687 |
| | 3.10 | 9.80 | ± | 0.80 | 0.150 | 6.04 | ± | 0.18 | 0.103 |
| | 6.30 | 10.34 | ± | 0.76 | 0.015 | 6.24 | ± | 0.13 | 0.561 |
| | 12.50 | 10.71 | ± | 0.61 | 0.001 | 6.23 | ± | 0.13 | 0.508 |
| | 25.00 | 10.67 | ± | 0.52 | 0.001 | 6.16 | ± | 0.20 | 0.324 |
| | 50.00 | 10.03 | ± | 0.86 | 0.070 | 6.35 | ± | 0.22 | 0.940 |
| | 100.00 | 9.69 | ± | 0.48 | 0.124 | 6.08 | ± | 0.21 | 0.166 |

| | | | | | | | | | |
|---|---|---|---|---|---|---|---|---|---|
| 1) PC : 25 µM VC-PMg 2) represents a significant difference for the cells not treated with test sample. Red letters : considerably increased | | | | | | | | | |

From the hydroxyapatite (calcined) of the Production Example 1, a significant activity of increasing a collagen production amount was observed. From the hydroxyapatite (non-calcined) of the Production Example 2 (reference), a significant increase in collagen production amount was observed in concentration range of 6.30 to 25.00 µg/mL, but the activity is weaker than the hydroxyapatite (calcined).

### <Application test on human epidermis>

Test condition: After taught with the purpose and procedures of the test, two subjects for the test received a patch test in the front side of their arms. After confirming no abnormality, three spots including a back of a hand, a lateral side of a neck, and an upper arm were visually examined and enlarged (enlargement ratio: × 200) photographic images thereof were taken using a microscope. Further, the cosmetic containing the hydroxyapatite (calcined) of the Production Example 1 at 1% concentration was applied for three continuous days, twice per day (i.e. morning and evening), 150 mg per each application on the area of which photographic image has been taken before starting the application. After the completion of the application on Day 3, the applied area was visually examined and enlarged (enlargement ratio: × 200) photographic images thereof were taken using a microscope. On Day 7 from starting the application, photographic images of the same area were also taken.

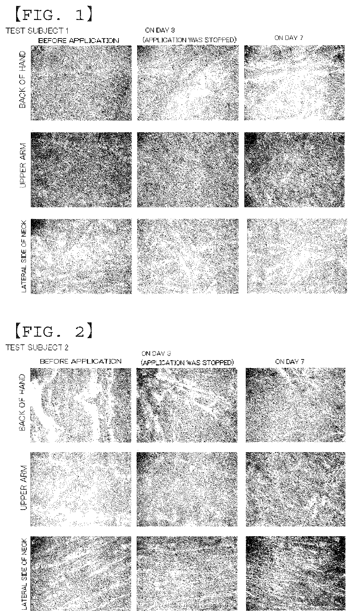

Before starting the application, no rising crista cutis was observed, and the skin was flat. However, on Day 3 from the application, it was found that the crista cutis slowly starts to show up, and the crista cutis was clearly visible. Further, on Day 7 from the application, the crista cutis was more clearly visible even though the application was performed for four days, and the skin was converted to more delicate skin compared to the skin before application. Therefore, it was found that the collagen production is enhanced. Appearance of the skin surface is shown in FIG. 1 and FIG. 2.

## Claims

1. Cosmetic use of a composition comprising
(i) calcium phosphate fine particles as effective ingredients, wherein
- the average particle diameter of the calcium phosphate fine particles is 20-250 nm,
- the calcium phosphate fine particles are a sintered body wherein the half width at d = 2.814, as measured by X ray diffraction (XRD), is ≤ 0.8, and
(ii) at least one material selected from alcohols, sugars, proteins, amino acids, water soluble vitamins, fat soluble vitamins, lipids, mucosaccharides, and an surface active agent,
for enhancing collagen production.

2. The use of a collagen production enhancer comprising
(i) calcium phosphate fine particles as effective ingredients, wherein
- the average particle diameter of the calcium phosphate fine particles is 20-250 nm,
- the calcium phosphate fine particles are a sintered body wherein the half width at d = 2.814, as measured by X ray diffraction (XRD), is ≤ 0.8, and
(ii) at least one material selected from alcohols, sugars, proteins, amino acids, water soluble vitamins, fat soluble vitamins, lipids, mucosaccharides, and an surface active agent,
as addendum for a cosmetic.

3. The use of claim 1 or 2, wherein the calcium phosphate fine particles are hydroxyapatite fine particles.

4. The use of any of claims 1-3, wherein the variation coefficient of the calcium phosphate fine particles is ≤ 20%.

5. The use of any of claims 1-4, wherein the sintered body is produced by a method including the steps of
- mixing primary particles containing calcium phosphate with a fusion preventive agent and,
- sintering the mixed particles obtained from the mixing step by exposing them to a sintering temperature in the range of 100-1,800°C.

## Patentansprüche

1. Kosmetische Verwendung einer Zusammensetzung, umfassend
(i) feine Calciumphosphatpartikel als wirksame Inhaltsstoffe, wobei
- der durchschnittliche Partikeldurchmesser der feinen Calciumphosphatpartikel 20-250 nm beträgt,
- die feinen Calicumphosphatpartikel ein Sinterkörper sind, wobei die Halbwertsbreite bei d = 2,814, gemessen durch Röntgenbeugung (XRD), ≤ 0,8 beträgt und
(ii) mindestens ein Material, ausgewählt aus Alkoholen, Zuckern, Proteinen, Aminosäuren, wasserlöslichen Vitaminen, fettlöslichen Vitaminen, Lipiden, Mucosacchariden und einer oberflächenaktiven Substanz,
zur Steigerung der Kollagenproduktion.

2. Verwendung eines Kollagenproduktion-steigernden Mittels, umfassend
(i) feine Calciumphosphatpartikel als wirksame Inhaltsstoffe, wobei
- der durchschnittliche Partikeldurchmesser der feinen Calciumphosphatpartikel 20-250 nm beträgt,
- die feinen Calicumphosphatpartikel ein Sinterkörper sind, wobei die Halbwertsbreite bei d = 2,814, gemessen durch Röntgenbeugung (XRD), ≤ 0,8 beträgt und
(ii) mindestens ein Material, ausgewählt aus Alkoholen, Zuckern, Proteinen, Aminosäuren, wasserlöslichen Vitaminen, fettlöslichen Vitaminen, Lipiden, Mucosacchariden und einer oberflächenaktiven Substanz,
als Zusatz für ein Kosmetikum.

3. Verwendung gemäß Anspruch 1 oder 2, wobei die feinen Calciumphosphatpartikel feine Hydroxyapatitpartikel sind.

4. Verwendung gemäß irgendeinem der Ansprüche 1-3, wobei der Variationskoeffizient der feinen Calicumphosphatpartikel ≤ 20 % beträgt.

5. Verwendung gemäß irgendeinem der Ansprüche 1-4, wobei der Sinterkörper durch ein Verfahren hergestellt wird, das die Schritte
- Mischen von Primärpartikeln, die Calciumphosphat enthalten, mit einem Verschmelzung-vorbeugenden Mittel und
- Sintern der gemischten Partikel, die aus dem Mischschritt erhalten werden, indem sie einer Sintertemperatur im Bereich von 100-1.800°C ausgesetzt werden.

## Revendications

1. Utilisation cosmétique d'une composition comprenant
(i) des particules fines de phosphate de calcium en tant qu'ingrédients actifs, dans laquelle
- le diamètre de particule moyen des particules fines de phosphate de calcium est de 20-250 nm,
- les particules fines de phosphate de calcium sont un corps fritté dans lequel la demi-largeur à d = 2.814, telle que mesurée par diffraction aux rayons X (DRX), est ≤ 0.8, et
(ii) au moins un matériau choisi parmi des alcools, sucres, protéines, acides aminés, vitamines solubles dans l'eau, vitamines solubles dans la graisse, lipides, muco-saccharides, et un agent actif de surface,
pour augmenter la production de collagène.

2. L'utilisation d'un exhausteur de production de collagène comprenant
(i) des particules fines de phosphate de calcium en tant qu'ingrédients actifs, dans laquelle
- le diamètre de particule moyen des particules fines de phosphate de calcium est de 20-250 nm,
- les particules fines de phosphate de calcium sont un corps fritté dans lequel la demi-largeur à d = 2.814, telle que mesurée par diffraction aux rayons X (DRX), est ≤ 0.8, et
(ii) au moins un matériau choisi parmi des alcools, sucres, protéines, acides aminés, vitamines solubles dans l'eau, vitamines solubles dans la graisse, lipides, muco-saccharides, et un agent actif de surface,
en tant qu'addendum pour un cosmétique.

3. L'utilisation de la revendication 1 ou 2, dans laquelle les particules fines de phosphate de calcium sont des particules fines d'hydroxyapatite.

4. L'utilisation de l'une quelconque des revendications 1-3, dans laquelle le coefficient de variation des particules fines de phosphate de calcium est ≤ 20%.

5. L'utilisation de l'une quelconque des revendications 1-4, dans laquelle le corps fritté est produit par une méthode comportant les étapes de
- le mélange de particules primaires contenant du phosphate de calcium avec un agent préventif de fusion, et
- le frittage des particules mélangées obtenues par l'étape de mélange en exposant celles-ci à une température de frittage dans un domaine de 100-1800°C.
